Europäisches Patentamt

European Patent Office

Office européen des brevets

(11) Veröffentlichungsnummer: **0 392 052**
**A1**

(12) # EUROPÄISCHE PATENTANMELDUNG

(21) Anmeldenummer: 89106528.6

(51) Int. Cl.5: **C07D 277/78**

(22) Anmeldetag: **12.04.89**

(43) Veröffentlichungstag der Anmeldung:
**17.10.90 Patentblatt 90/42**

(84) Benannte Vertragsstaaten:
**DE FR GB**

(71) Anmelder: **DOLNOSLASKIE ZAKLADY CHEMICZNE " ORGANIKA" Przedsiebiorstwo Panstwowe P-58-130 Zarow woj. Walbrzych(PL)**

(72) Erfinder: **Grzybowska, Danuta**
**ul. Klonowa 17**
**P-58-100 Swidnica(PL)**
Erfinder: **Serwatka, Jerzy**
**ul. Zamenhofa 49/27**
**P-58-105 Swidnica(PL)**
Erfinder: **Wiecek, Jerzy**
**ul. Rogowska 84/3**
**P-54-440 Wroclaw(PL)**
Erfinder: **Bryk, Danuta**
**ul. Armii Czerwonej 43/8**
**P-58-130 Zarow(PL)**

(74) Vertreter: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) Verfahren zur Herstellung von 2,2'-Benzothiazyldisulfid.

(57) Die Erfindung betrifft die Herstellung von 2,2'-Benzothiazyldisulfid auf dem Wege der Oxidation von 2-Merkaptobenzothiazol im wässrigen Medium. Die Reaktion wird unter Verwendung der von N-Chlorderivaten der primären oder sekundären Amine als Oxidationsmittel durchgeführt.

Es ist empfehlenswert, 2-Merkaptobenzothiazol in einer wässrigen Suspension mit dem pH-Wert 7-9 zu oxidieren.

EP 0 392 052 A1

## Verfahren zur Herstellung von 2,2′-Benzothiazyldisulfid

Der Gegenstand der Erfindung betrifft die Herstellung von 2,2′-Benzothiazyldisulfid, das als Mittel für die Beschleunigung der Kautschukvulkanisation verwendet wird.

Im Stand der Technik ist die Herstellung von 2,2′-Benzothiazyldisulfid auf dem Wege der Oxidation von 2-Merkaptobenzothiazol unter Verwendung der folgenden Oxidationsmittel bekannt: Salpeter- und salpetrige Säure, Stickoxide, Luftsauerstoff, Wasserstoffsuperoxid, Dichromate, Persulfate, Kaliumpermanganat, Chlor, Chlorate, Hypochlorite, Nitrosilchlorid, Chlorstickstoff. Auch die elektrolytische Oxidation ist geläufig. Die Oxidation mit der Salpetersäure und die Nitritmethoden führen zur Entstehung von grossen Mengen unerwünschter Nebenprodukte, insbesondere Stickoxide, Mineralsalze, Produkte der Überoxidation von 2-Merkaptobenzothiazol. Das auf diese Art und Weise gewonnene Produkt ist bräunlich statt weiss oder cremefarbig. Die helle Farbe ermöglicht, diesen Beschleuniger in einer grossen Vielfalt von Farben anzuwenden. Die Oxidation mit dem Oxid erfordert die Anwesenheit der Katalysatoren, deren industrielle Gewinnung und Verwendung nicht einfach ist. Die Gasoxidationsmittel wie Stickoxide, Luftsauerstoff und Chlor erfordern die Verwendung komplizierter Anlagen, die die effektive Gasmischung mit der Flüssigkeit sichern, wobei ein Produkt in Form einer schaumhaltigen, schwer filtrierbaren Suspension entsteht. Bei der Gewinnung von 2,2′-Benzothiazyldisulfid werden die Reaktionsmittel in gelöster Form angewandt, was zur Entstehung der postreaktiven Massesuspension führt. Das Produkt fällt in nicht grösseren Mengen als 20% an. Einige der bekannten Methoden setzen die Anwendung von organischen, brennbaren, gebrauchsheiklen Lösungsmitteln voraus.

Ein wesentlicher Nachteil der bekannten Gewinnungsarten ist die Tatsache, dass kein technisches Produkt mit hohem 2,2′-Benzothiazyldisulfidgehalt gewonnen wird. Andererseits erfordert die Gewinung eines hochsauberen Produkts viele technische, für den industriellen Gebrauch anwendungsschwere Mittel. Eine besonders kritische Verunreinigung von 2,2′-Benzothiazyldisulfid ist 2-Merkaptobenzothiazol - ein Beschleunigungsmittel für die Kautschukvulkanisation. 2-Merkaptobenzothiazol beeinflusst, wie andere Beschleuniger, den Verlauf des Kautschukvulkanisationsprozesses und stört den Vulkanisationsverlauf, wenn es eine Verunreinigung von technischem 2,2′-Benzothiazyldisulfid darstellt. Auf diese Art und Weise hat es einen negativen Einfluss auf die Eigenschaften der Gummierzeugnisse.

Für die Anwesenheit von 2-Merkaptobenzothiazol im technischen 2,2′-Benzothiazyldisulfid ist der pH-Wert des Reaktionsmediums verantwortlich. Bei den bekannten Herstellungsweisen wird die Oxidation mit oxidativen Säuren oder Oxidationsmitteln durchgeführt, die eine Säuerung der Reaktionsmasse bewirken. Die Chloroxidation wird von Nebenreaktionen begleitet, die mit dem Entstehen von Chlorwasserstoff verbunden sind. Unter diesen Bedingungen treten die Erscheinungen der übermässigen pH-Wertsenkung des Reaktionsmediums auf. Dies erschwert die Überreaktion von 2-Merkaptobenzothiazol und führt zur Gewinnung eines mit diesem Reaktionssubstrat verunreinigten Produktes.

Unter allen bekannten Arten der Herstellung von 2,2′-Benzothiazyldisulfid werden die Methoden der Chloroxidation allgemein verwendet. Sie bilden das Thema der neuesten Patente. Aus der US-Patentschrift Nr. 4 482 720 ist eine Gewinnung von 2,2′-Benzothiazyldisulfid bekannt, die auf der Oxidation des Alkalimetallsalzes von 2-Merkaptobenzothiazol mit Chlorgas bei konstanter Einhaltung des pH-Wertes im Bereich von 6-9 (optimal 7-8,5) sowie auf der Einhaltung des Wertes des Reduktions-Oxidationspotentials von -50 zu +100 mV (optimal 5-25 mV) beruht. Die Reaktionsmasse wird dabei stark gemischt. Um eine hohe Prozessleistung und eine hohe Qualität des Produkts zu gewinnen, sollten die Parameter in einem engen Bereich um die optimalen Werte konstant gehalten werden. Im industriellen Einsatz ist dies sehr schwer zu verwirklichen.

Die Erfindung bezieht sich auf die Herstellung von 2,2′-Benzothiazyldisulfid auf dem Wege der Oxidation der wässrigen Suspension von 2-Merkaptobenzothiazol. Dann wird das Produkt abfiltriert, gewaschen und getrocknet. Das Wesen der Erfindung besteht darin, dass N-Chlorderivate der primären oder sekundären Amine als Oxidationsmittel verwendet werden, wobei im Prozess die im Reaktionsmedium oder in einem anderen Stadium gewonnenen N-Chloramine verwendet werden. Es ist nützlich, die Suspension von 2-Merkaptobenzothiazol bei einem pH-Wert von 7-9 zu oxidieren.

Es wurde gefunden, dass bei der Oxidation von 2-Merkaptobenzothiazol mit N-Chloraminen ein Produkt von sehr vorteilhaften Parametern gewonnen wird. Gemäss der Erfindung bleibt der pH-Wert des Reaktionsmediums auf dem Niveau des Anfangswertes ohne die Notwendigkeit irgendeiner Regulierung während der Synthese. Dieser Erfolg tritt durch die Anwendung von N-Chloraminen als Oxidationsmittel auf. Bei der Oxidation von 2 Molen von 2-Merkaptobenzothianol mit einem Mol von N-Chloramin gewinnt man 1 Mol von 2,2′-Benzothiazyldisulfid, 1 Mol von Amin und 1 Mol von Chlorwasserstoff. Auf diese Art und Weise entsteht, zusammen mit dem Chlorwasserstoff, ein freies Amin, das als Säureakzeptor wirkt. Die Anwendung der N-Chloramine als Oxidationsmittel bewahrt die Reaktionsmasse vor der gefährlichen pH-Wertsenkung, was die Gewinnung eines mit 2-Merkaptobenzothiazol minimal verunreinigten Produktes zur Folge hat. Das Verfahren ist sehr einfach und wirksam. Sehr dicke Suspensionen können effektiv bis zu 50% 2-Merkaptobenzothiazolgehalt oxidiert werden. Das Verfahren wird durch eine geringe Ablaufmenge gekennzeichnet. Die Amine können einfach wiedergewonnen und wiederverwendet werden. Die hohe Konzentration des Reaktionsmediums ermöglicht eine grosse Produktionsleistungfähigkeit der Anlagen. Die Reaktion wird im Wassermedium geführt, was die Filtration und die Produkttrocknung erleichtert. Der Oxidationsprozess verläuft effektiv sogar unter Verwendung von typischen, langsamdrehenden Rührern, da die Oxidationsmittel gute Dispergierflüssigkeiten im Reaktionsmedium sind. Die Anlage ist bedienungsleicht und setzt keine automatischen Steuerungs- und Regulierungssysteme voraus. Im Prozess entstehen keine umweltfeindlichen Nebenprodukte.

Die Erfindung ist in drei Herstellungsbeispielen dargestellt.

## Beispiel I

Das Beispiel bezieht sich auf die Verwendung von 2-Merkaptobenzothiazol in einer festen Form und von N-Chloramin, das in einem gesondertem Stadium gewonnen wird. 1250 dm³ Wasser wird zu einem Reaktor mit der Kapazität von 3 m³ dosiert. Der Reaktor ist mit einem Rührer, einem Kühlmantel, einem Thermometer und einem Elektrodensystem zur pH-Messung ausgestattet. Der Rührer wird in Betrieb gesetzt, 500 kg von trockenem 2-Merkaptobenzothiazol werden durch ein Schnekkendosiergerät eingeführt, und eine wässerige Lösung von Natriumhydroxid mit einer Konzentration von 10% wird in einer zur pH-Werteinstellung des Reaktorinhalts auf einen Wert von 8-9 notwendigen Menge zugetropft. Der Reaktorinhalt wird ca. 30 min gerührt, dann werden 236 dm³ von N-Chlordiisopropyloamin in ungefähr 2 Stunden zudosiert, wobei die Temperatur im Reaktor bei 35°C gehalten wird. Die gewonnene Produktsuspension wird 30 min gerührt, filtriert, gewaschen und getrocknet. 495 kg von technischem 2,2'-Benzothiazyldisulfid werden in Form eines cremefarbigen Pulvers mit einer Anfangsschmelztemperatur von 176°C und einer Reinheit von 99,0% gewonnen.

## Beispiel II

Das Beispiel bezieht sich auf die Verwendung von 2-Merkaptobenzothiazol in Form einer wässrigen Lösung des Natriumsalzes und von N-Chloramin, das in einem gesonderten Stadium erhalten wird. 1465 dm³ der wässrigen Lösung des Natriumsalzes von 2-Merkaptobenzothiazol mit einem Gehalt von 140 g 2-Merkaptobenzothiazol in 1 dm³ werden in einen Reaktor mit der Kapazität von 3 m³ eingeführt. Der Reaktor ist mit einem Rührer, einem Kühlmantel, einem Thermometer und einem Elektrodensystem zur pH-Messung ausgestattet. Der Rührer wird in Betrieb gesetzt, dann wird die 20%-ige Schwefelsäure in einer zur pH-Werteinstellung des Reaktorinhaltes auf einen Wert von 8-9 notwendigen Menge zudosiert. Der Reaktorinhalt wird ca. 30 min gerührt, dann werden 98 dm³ von N-Chlorodiisopropylamin in etwa einer Stunde zudosiert, wobei die Temperatur der Reaktionsmasse bei 35°C gehalten wird. Die gewonnene Produktsuspension wird 30 min gerührt, dann filtriert, gewaschen und getrocknet. 203 kg von technischem 2,2'-Benzothiazyldisulfid werden in Form eines cremefarbigen Pulvers mit einer Anfangsschmelztemperatur von 175°C und einer Reinheit von 99,0% gewonnen.

## Beispiel III

Das Beispiel bezieht sich auf die Verwendung von 2-Merkaptobenzothiazol in Form einer wässrigen Lösung des Natriumsalzes und von N-Chloramin, das im Reaktionsmedium gewonnen wird. 1264 dm³ der wässrigen Natriumsalzlösung von 2-Merkaptobenzothiazol mit einem Gehalt von 140 g von 2-Merkaptobenzothiazol in 1 dm³ werden in einen Reaktor mit der Kapazität von 3 m³ eingeführt. Der Reaktor ist mit einem Rührer, einem Kühlmantel, einem Thermometer und einem Elektrodensystem zur pH-Messung ausgestattet. Der Rührer wird in Betrieb gesetzt, 61 dm³ von Cyclohexylamin werden zudosiert, dann wird die 20%-ige Schwefelsäure in einer zur pH-Werteinstellung des Reaktionsinhaltes auf einen Wert von 7-9 notwendigen Menge zudosiert. Der Reaktorinhalt wird 30 min gerührt, dann werden 359 dm³ von wässrigem Natriumhypochlorit mit einem Gehalt von 160 g an aktivem Chlor in 1 dm³ während 2 Stunden zugegeben, wobei die Temperatur der Reaktionsmasse bei 35°C gehalten wird. Die gewonnene Produktsuspension wird 30 min gerührt, dann filtriert, gewaschen und getrocknet. 172 kg von technischem 2,2'-Benzothiazyldisulfid werden in Form eines cremefarbigen Pulvers mit der Anfangsschmelztemperatur von 173°C und der Reinheit von 98,0% gewonnen.

**Ansprüche**

1. Verfahren zur Herstellung von 2,2$'$-Benzothiazyldisulfid auf dem Wege der Oxidation von 2-Merkaptobenzothiazol im wässrigen Medium, dadurch gekennzeichnet, dass N-Chlorderivate der primären oder sekundären Amine als Oxidationsmittel verwendet werden.

2. Verfahren nach Anspruch 1, dadurch gekennzeichnet, dass die wässrige Suspension von 2-Merkaptobenzothiazol bei einem pH-Wert von 7-9 oxidiert wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-3 463 783 (R. STRAUSS)<br>* Insgesamt *<br>--- | 1,2 | C 07 D 277/78 |
| A | US-A-2 265 319 (M.G. SHEPARD)<br>* Insgesamt *<br>--- | 1,2 | |
| A | EP-A-0 112 794 (THE GOODYEAR TIRE AND RUBBER CO.)<br>* Ansprüche *<br>----- | 1,2 | |
| | | | RECHERCHIERTE SACHGEBIETE (Int. Cl.5)<br><br>C 07 D 277/00 |

Der vorliegende Recherchenbericht wurde für alle Patentansprüche erstellt

| Recherchenort | Abschlußdatum der Recherche | Prüfer |
|---|---|---|
| DEN HAAG | 13-11-1989 | HENRY J.C. |